(19) Europäisches Patentamt / European Patent Office / Office européen des brevets

(11) **EP 2 759 247 A1**

(12) **EUROPEAN PATENT APPLICATION**
published in accordance with Art. 153(4) EPC

(43) Date of publication:
**30.07.2014 Bulletin 2014/31**

(51) Int Cl.:
**A61B 1/00** *(2006.01)* **G01N 21/27** *(2006.01)*

(21) Application number: **12834091.6**

(22) Date of filing: **19.09.2012**

(86) International application number:
**PCT/JP2012/073948**

(87) International publication number:
**WO 2013/042690 (28.03.2013 Gazette 2013/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2011 US 201161536705 P**

(71) Applicant: **Olympus Corporation**
**Shibuya-ku**
**Tokyo 151-0072 (JP)**

(72) Inventors:
• **TAKAOKA, Hideyuki**
  **Tokyo 151-0072 (JP)**
• **ITO, Ryosuke**
  **Tokyo 151-0072 (JP)**

(74) Representative: **Schicker, Silvia**
**Wuesthoff & Wuesthoff**
**Patent- und Rechtsanwälte**
**Schweigerstrasse 2**
**81541 München (DE)**

(54) **OPTICAL MEASUREMENT APPARATUS AND CALIBRATION METHOD**

(57)    An optical measurement apparatus and a calibration method, which are able to readily perform a calibration process, are provided. A recording unit 26 that records a value of a ratio of a first detection intensity when illumination light is irradiated by a measurement probe 3 to a first medium having the same refractive index as a specimen S1 to a second detection intensity when the illumination light is irradiated by the measurement probe 3 to a second medium, and a calculation unit 271 that calculates a background intensity of background light to be used in correcting returned light of the illumination light from the specimen S1, from a product of the value of the ratio recorded in the recording unit 26 and a third detection intensity when the illumination light is irradiated by the measurement probe 3 to the second medium.

FIG.1

EP 2 759 247 A1

**Description**

Field

The present invention relates to an optical measurement apparatus that irradiates body tissue with illumination light and estimates, based on measurement values of returned light of the illumination light reflected and/or scattered from the body tissue, characteristics of the body tissue, and to a calibration method executed by the optical measurement apparatus. Background

Recently, optical measurement apparatuses are known, which irradiate body tissue with illumination light and estimate, based on measurement values of returned light of the illumination light reflected or scattered from the body tissue, characteristics of the body tissue. An optical measurement apparatus is used in combination with an endoscope for observing an organ such as a digestive organ. As such an optical measurement apparatus, an optical measurement apparatus has been proposed, which uses low-coherence enhanced backscattering spectroscopy (LEBS) to detect characteristics of body tissue by irradiating low-coherence white light of a short spatial coherence length from a distal end of an illumination fiber of a measurement probe and measuring, using a plurality of detection fibers, an intensity distribution of scattered light beams of plural angles (see Patent Literature 1).

Citation List

Patent Literature

Patent Literature 1: U. S. Patent Application Publication No. 2009/0009759

Summary

Technical Problem

The above described measurement values of the returned light of the illumination light from the body tissue include a component of background light (noise), which is the illumination light reflected and/or scattered inside the measurement probe, and a component of background light, which is the illumination light reflected at a boundary plane between the measurement probe and the body tissue due to a difference in refractive indexes between a material of the measurement probe and the body tissue when measurement is performed by contacting the measurement probe with the body tissue. Therefore, the above described optical measurement apparatus contacts a substance for calibration closely to the distal end of the measurement probe to measure background intensity corresponding to the components of background light and removes the components of background light using the background intensity. This substance for calibration needs to be a liquid, such as water, which has a refractive index close to that of the body tissue.

However, for the above optical measurement apparatus, for every measurement, the background intensity need to be measured and a calibration process needs to be performed using the background intensity, and thus complicated operations have been a burden on users.

The present invention has been made in view of the above, and an object of the invention is to provide an optical measurement apparatus and a calibration method that are able to readily perform a calibration process. Solution to Problem

To solve the above-mentioned problem and achieve the object, an optical measurement apparatus according to the invention includes: a measurement probe that irradiates a specimen with illumination light and optically receives returned light of the illumination light reflected and/or scattered from the specimen; a detection unit that detects the returned light optically received by the measurement probe; a recording unit that records therein a value of a ratio of a first detection intensity to a second detection intensity, the first detection intensity being an intensity of the returned light detected when a first medium having a refractive index that is the same as that of the specimen is irradiated with the illumination light by the measurement probe and detected in a state of not including the returned light from the first medium, and the second detection intensity being an intensity of the returned light detected when a second medium is irradiated with the illumination light by the measurement probe and detected in a state of not including the returned light from the second medium; and a calculation unit that calculates, from a product of a third detection intensity and the value of the ratio, a background intensity of background light to be used in correcting the returned light from the specimen, the third detection intensity being an intensity of the returned light detected when the second medium is irradiated with the illumination light by the measurement probe and detected in a state of not including the returned light from the second medium.

According to the optical measurement apparatus of the invention, in the above invention, the calculation unit subtracts the background intensity from a detection intensity of the returned light from the specimen detected by the detection unit when the measurement probe irradiates the specimen with the illumination light.

According to the optical measurement apparatus of the invention, in the above invention, the detection unit has a spectrometer, and the first detection intensity, the second detection intensity, and the third detection intensity are values detected for each wavelength spectrally dispersed by the spectrometer.

According to the optical measurement apparatus of the invention, in the above invention, the first medium is water.

According to the optical measurement apparatus of the invention, in the above invention, the second medium is air.

According to the optical measurement apparatus of the invention, in the above invention, the measurement probe is detachable to a main body unit of the optical measurement apparatus.

According to the optical measurement apparatus of the invention, in the above invention, the specimen is body tissue.

According to the optical measurement apparatus of the invention, in the above invention, an absolute value of a difference between the refractive index of the specimen and the refractive index of the first medium is equal to or less than 0.1.

According to the optical measurement apparatus of the invention, in the above invention, the first detection intensity is measured with respect to the first medium accommodated in a container provided with, on an inner surface thereof, a light absorption member that absorbs light, and the second detection intensity is measured with respect to the second medium accommodated in the container.

A calibration method according to the invention is executed by an optical measurement apparatus that includes a measurement probe, which irradiates a specimen with illumination light and optically receives returned light of the illumination light reflected and/or scattered from the specimen, and that performs optical measurement of the specimen. The calibration method includes the steps of: calculating a background intensity of background light to be used in correcting the returned light from the specimen, from a product of a value and a third detection intensity, the value being of a ratio of a first detection intensity to a second detection intensity, the first detection intensity being an intensity of the returned light detected when a first medium having a refractive index that is the same as that of the specimen is irradiated with the illumination light by the measurement probe and detected in a state of not including the returned light from the first medium, the second detection intensity being an intensity of the returned light detected when a second medium is irradiated with the illumination light by the measurement probe and detected in a state of not including the returned light from the second medium, and the third detection intensity being an intensity of the returned light detected when the second medium is irradiated with the illumination light by the measurement probe and detected in a state of not including the returned light from the second medium; and subtracting the background intensity from a detection intensity of the returned light from the specimen.

Advantageous Effects of Invention

According to the present invention, a calculation unit calculates a background intensity of background light, from a product of a third detection intensity and a value of a ratio of a first detection intensity to a second detection intensity, the value being recorded in a recording unit and the background intensity being used when returned light of illumination light from a specimen is corrected. Accordingly, measurement of the background light using water or a liquid upon measurement of body tissue is omittable, and thus an effect of being able to facilitate operations of a calibration process involved in the measurement is demonstrated.

Brief Description of Drawings

FIG. 1 is a block diagram schematically illustrating a configuration of an optical measurement apparatus according to a first embodiment of the present invention.

FIG. 2 is a diagram schematically illustrating a cross section of a distal end of a measurement probe of the optical measurement apparatus according to the first embodiment of the present invention, the cross section being but along a longitudinal direction thereof.

FIG. 3 is a front view of FIG. 1 from a direction of an arrow A.

FIG. 4 is a diagram illustrating a situation in which the optical measurement apparatus according to the first embodiment of the present invention is used in an endoscopic system.

FIG. 5 is a diagram schematically illustrating a first measurement process performed as a calibration process by the optical measurement apparatus according to the first embodiment of the present invention.

FIG. 6 is a diagram schematically illustrating a second measurement process performed as a calibration process by the optical measurement apparatus according to the first embodiment of the present invention.

FIG. 7 is a diagram schematically illustrating a third measurement process performed as a calibration process by the optical measurement apparatus according to the first embodiment of the present invention before measurement of a specimen.

FIG. 8 is a diagram schematically illustrating a measurement process of a specimen performed by the optical measurement apparatus according to the first embodiment of the present invention.

FIG. 9 is diagram illustrating a relation between incident light intensity incident on the measurement probe of the optical measurement apparatus according to the first embodiment of the present invention and signal intensity.

FIG. 10 is a block diagram illustrating a configuration of an optical measurement apparatus according to a second embodiment of the present invention.

FIG. 11 is a block diagram illustrating a configuration of an optical measurement apparatus according to a third embodiment of the present invention.

FIG. 12 is a diagram schematically illustrating a cross section of a distal end of a measurement probe of the optical measurement apparatus according to the third embodiment of the present invention, the cross section being cut along a longitudinal direction thereof.

FIG. 13 is a diagram illustrating a relation between a measurement spectrum when the optical measurement apparatus according to the third embodiment of the present invention measures a specimen and a back ground light spectrum.

FIG. 14 is a diagram schematically illustrating a third measurement process performed as a calibration process by an optical measurement apparatus according to another embodiment of the present invention.

Description of Embodiments

Hereinafter, with reference to the drawings, preferable embodiments of an optical measurement apparatus according to the present invention will be described in detail. Further, in describing the drawings, the same portions are appended with the same reference signs. Further, the drawings are schematic, and it is to be noted that the relation between the thickness and width of each component and the ratios among the respective components are different from the actual. Further, a portion is included, which has different size relations and ratios among the drawings. The present invention is not limited by the embodiments.

(First Embodiment)

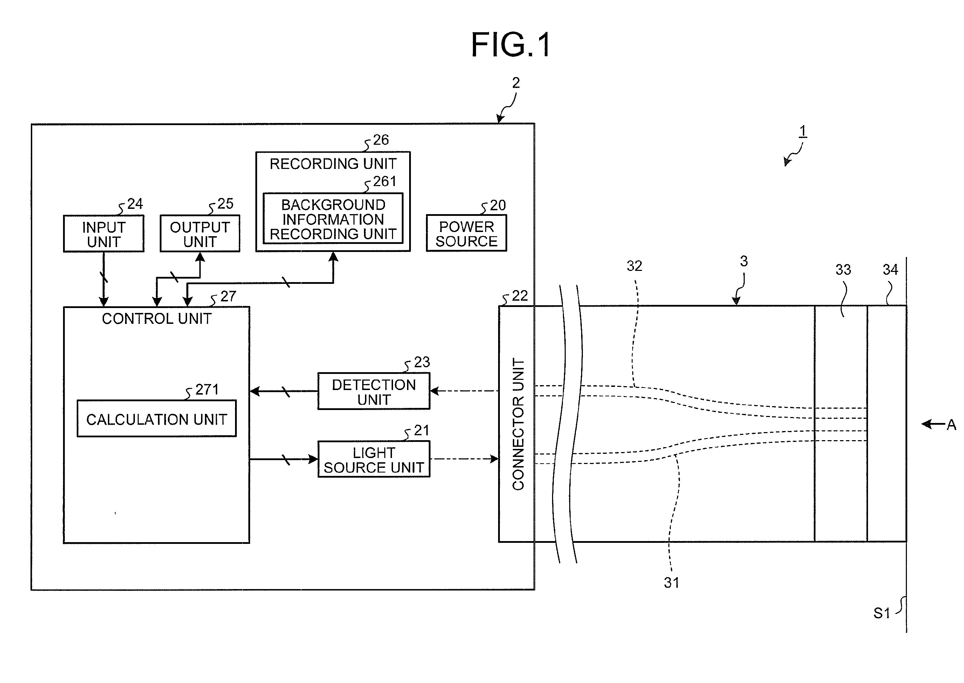

FIG. 1 is a block diagram that schematically illustrates a configuration of an optical measurement apparatus according to a first embodiment of the present invention.

An optical measurement apparatus 1 illustrated in FIG. 1 includes a main body unit 2, which performs optical measurement with respect to a specimen S1 such as body tissue that is a scatterer, and a measurement probe 3 of a disposable type, which is detachable with respect to the main body unit 2 and inserted into a subject.

First, the main body unit 2 is described. The main body unit 2 includes a power source 20, a light source unit 21, a connector unit 22, a detection unit 23, an input unit 24, an output unit 25, a recording unit 26, and a control unit 27. The power source 20 supplies electric power to each component of the main body unit 2.

The light source unit 21 irradiates to the measurement probe 3, via the connector unit 22, illumination light of incoherent light, which is to be irradiated to the specimen S1. The light source unit 21 is realized by using an incoherent light source such as a white light emitting diode (LED), a xenon lamp, a tungsten lamp, and a halogen lamp, and a plurality of lenses. Examples of such lenses include condenser lenses and collimator lenses. The light source unit 21 irradiates the illumination light having a wavelength component included in a specified wavelength band.

To the connector unit 22, the measurement probe 3 is detachably connected. The connector unit 22 propagates the illumination light irradiated by the light source unit 21 to the measurement probe 3 and propagates to the detection unit 23 the light that has entered from the measurement probe 3.

The detection unit 23 detects returned light of the illumination light that has been irradiated from the measurement probe 3 and reflected and/or scattered from the specimen S1 and outputs a result of this detection to the control unit 27. Specifically, the detection unit 23 detects spectrum components and an intensity distribution of scattered light that has entered from the measurement probe 3 and outputs results of this detection to the control unit 27. The detection unit 23 is realized by using a spectrometer, a light receiving sensor, or the like.

The input unit 24 receives, and outputs to the control unit 27, input of an instruction signal instructing activation of the main body unit 2, an instruction signal instructing start of measurement of the specimen S1 by the main body unit 2, an instruction signal instructing a calibration process, and the like. The input unit 24 is realized by using a push-type switch, a touch panel, or the like.

The output unit 25 outputs, under control by the control unit 27, various information in the main body unit 2, for example, measurement results of the specimen S1. The output unit 25 is realized by using a display of a liquid crystal, organic electroluminescence (EL), or the like, and a speaker or the like.

The recording unit 26 is realized by using a volatile memory, a non-volatile memory, and the like, and records therein various programs for operating the main body unit 2, various data and various parameters to be used in an optical measurement process, and the like. Further, the recording unit 26 records therein the measurement results of the specimen S1 by the main body unit 2. Further, the recording unit 26 has a background information recording unit 261.

The background information recording unit 261 records a value of a ratio of a first detection intensity to a second detection intensity, the first detection intensity being an intensity of returned light detected by the detection unit 23 when the illumination light is irradiated by the measurement probe 3 to a first medium having the same refractive index as the body tissue and detected in a state of not including returned light of the illumination light from the first medium, and the second detection intensity being an intensity of returned light detected by the detection unit 23 when the illumination light is irradiated by the measurement probe 3 to a second medium and detected in a state of not including returned light of the illumination light from the second medium. Herein, the first medium is water, ethanol, or silicone oil. Further, the second medium is air. Further, the value of the ratio is a value obtained by dividing the first detection intensity by the second detection intensity.

The control unit 27 comprehensively controls the main body unit 2 by carrying out transfer or the like of instruction information or data corresponding to each component of the main body unit 2. The control unit 27 is configured by using a central processing unit (CPU) or the like. The control unit 27 has a calculation unit 271.

The calculation unit 271 calculates an intensity of background light to be used in correcting the returned light of the illumination light from the specimen S1, from a product of a third detection intensity and the value of the ratio of the first detection intensity to the second detection intensity, the third detection intensity being an intensity of returned light detected by the detection unit 23 when the illumination light is irradiated by the measurement probe 3 to the second medium and detected in a state of not including the returned light of the illumination light from the second medium and the value of the ratio being recorded in the background information recording unit 261. Further, the calculation unit 271 subtracts the background intensity of background light (hereinafter, "background intensity") from the detection intensity of the returned light of the illumination light from the specimen S1 detected by the detection unit 23 when the measurement probe 3 irradiates the illumination light to the specimen S1.

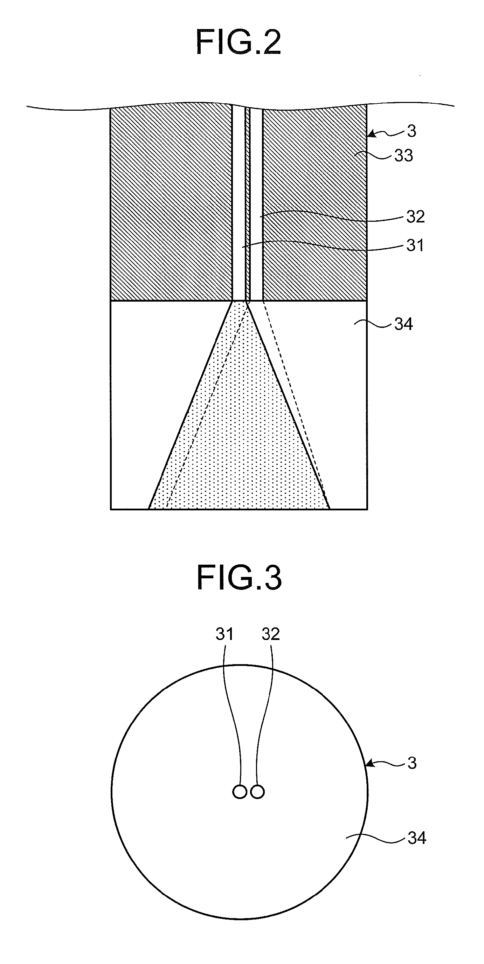

Next, the measurement probe 3 is described. FIG. 2 is a diagram that schematically illustrates a cross section of a distal end of the measurement probe 3, the cross section being cut along a longitudinal direction thereof. FIG. 3 is a front view of FIG. 1 from a direction of an arrow A.

The measurement probe 3 illustrated in FIGS. 1 to 3 includes an illumination fiber 31, a detection fiber 32, a fiber holding portion 33, and a rod lens 34 (optical element).

The illumination fiber 31 is realized by using an optical fiber, and irradiates to the specimen S1, via the rod lens 34, the illumination light entering, via the connector unit 22, from the light source unit 21.

The detection fiber 32 is realized by using an optical fiber, and detects (optically receives) via the rod lens 34 and propagates to the detection unit 23, the returned light of the illumination light reflected and/or scattered from the specimen S1.

The fiber holding portion 33 holds each of distal ends of the illumination fiber 31 and detection fiber 32 to be arranged in a straight line. Specifically, the fiber holding portion 33 holds them such that optical axes of the illumination fiber 31 and detection fiber 32 become parallel to each other. The fiber holding portion 33 is realized by using a glass, a resin, a metal, or the like.

The rod lens 34 is provided on a distal end of the fiber holding portion 33. The rod lens 34 is realized by using a glass, a plastic, or the like having a specified transmissivity, and is columnar such that a distance from respective distal ends of the illumination fiber 31 and detection fiber 32 to the specimen S1 becomes constant.

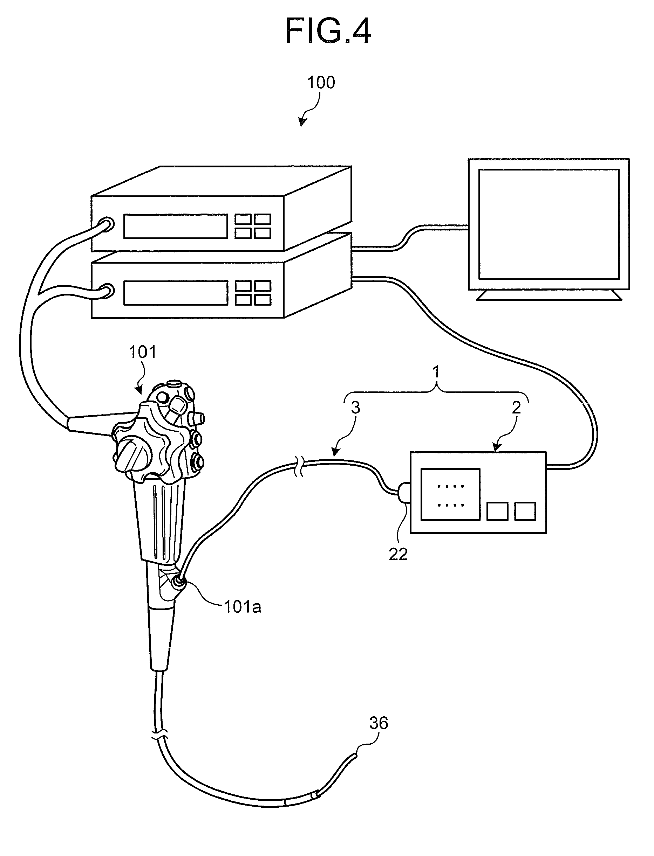

In the optical measurement apparatus 1 configured as above, as illustrated in FIG. 4, the measurement probe 3 is inserted into a subject via a treatment tool channel 101a provided in an endoscopic apparatus 101 (endoscope) of an endoscopic system 100, the illumination fiber 31 irradiates the illumination light to the specimen S1, and the detection fiber 32 detects and propagates to the detection unit 23, the returned light of the illumination light reflected and/or scattered from the specimen S1. Thereafter, the calculation unit 271 calculates characteristic values on characteristics of the specimen S1, based on detection results detected by the detection unit 23 and background information recorded in the background information recording unit 261.

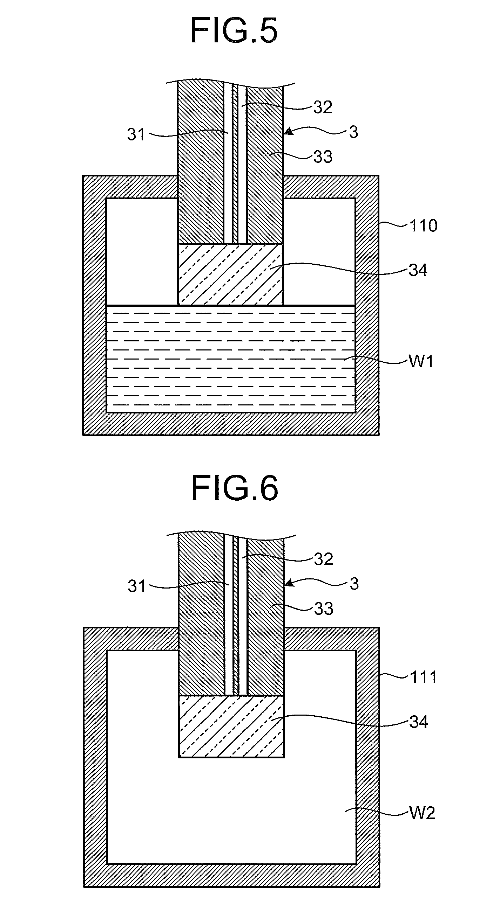

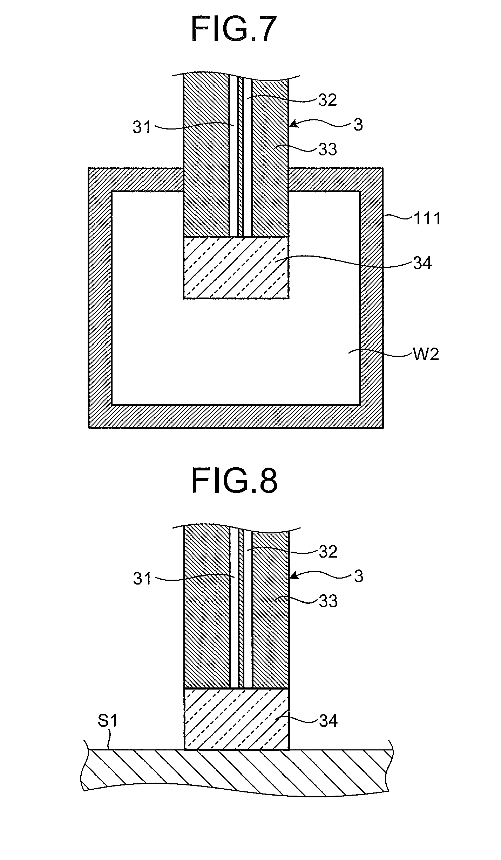

Next, a calculation process including a calibration process of the optical measurement apparatus 1 is described. FIG. 5 is a diagram that schematically illustrates a first measurement process performed as the calibration process by the optical measurement apparatus 1. FIG. 6 is a diagram that schematically illustrates a second measurement process performed as the calibration process by the optical measurement apparatus 1. FIG. 7 is a diagram that schematically illustrates a third measurement process performed as the calibration process by the optical measurement apparatus 1 before measurement of the specimen S1. FIG. 8 is a diagram that schematically illustrates a measurement process of the specimen S1 performed by the optical measurement apparatus 1.

First, the first measurement process is described. As illustrated in FIG. 5, the optical measurement apparatus 1 performs the first measurement process of inserting the distal end of the measurement probe 3 into a container 110 in which a first medium W1 having a refractive index close to that of the specimen S1 is accommodated. A refractive index of a biological cell is approximately 1.35 to 1.38 in a visible wavelength range (for example, see Phys. Med. Biol. 41, 369 (1996), or Opt. Lett. 31, 2759 (2006)). The first medium W1 is a transparent substance that doe not cause scattering. Specifically, when the optical measurement apparatus 1 measures body tissue as the specimen S1, water is used. The refractive index of water is approximately 1.33. As the first medium W1, ethanol (refractive index of approximately 1.36), silicone oil (refractive index of approximately 1.41), or the like may be used.

The container 110 is provided with, on an inner surface thereof, a member that does not reflect light or a light absorption member that absorbs wavelength of light irradiated by the light source unit 21. Specifically, the inner surface of the container 110 is coated in black. Further, a diameter of an insertion opening 110a of the container 110 is of a size that is approximately the same as that of a diameter of the measurement probe 3.

Accordingly, the illumination light irradiated by the illumination fiber 31 is absorbed within the container 110 and does not enter the detection fiber 32. Further, light does not enter from the outside of the container 110. As a result, the detection unit 23 is able to detect the background light generated inside the detection fiber 32. The detection unit 23 outputs a signal intensity "a" (first detection intensity "a") detected, to the background information recording unit 261 of the recording unit 26.

Next, the second measurement process is described. As illustrated in FIG. 6, the optical measurement apparatus 1 performs the second measurement process of inserting the distal end of the measurement probe 3 into a container 111, in which a second medium W2 is accommodated. The second medium W2 is air. As the second medium W2, nitrogen or the like may be used.

The container 111 is provided with, on an inner surface thereof, a member that does not reflect light or a light absorption member that absorbs wavelength of light irradiated by the light source unit 21. Specifically, the inner surface of the container 111 is coated in black. Further, a diameter of an insertion opening 111a of the container 111 is of a size that is approximately the same as that of a diameter of the measurement probe 3. As the container 111, one that is similar to the container 110 may be used.

Accordingly, the illumination light irradiated by the illumination fiber 31 is absorbed within the container 111, and does not enter the detection fiber 32. Further, light does not enter from the outside of the container 111. As a result, the detection unit 23 is able to detect the background light generated inside the detection fiber 32. The detection unit 23 outputs a signal intensity "b" (second detection intensity "b") detected, to the background information recording unit 261 of the recording unit 26. Further, the second measurement process performed by the optical measurement apparatus 1 is preferably performed within a specified time period after the first measurement process is performed. Accordingly, without variation or the like in the intensity of the illumination light irradiated by the light source unit 21, the first measurement process and the second measurement process are able to be performed under approximately the same conditions with light of the same intensity. The optical measurement apparatus 1 only needs to measure the first measurement process and the second measurement process once. Therefore, the optical measurement apparatus 1 only needs to perform the first measurement process and the second measurement process at the time of shipment thereof.

Next, the third measurement process is described. As illustrated in FIG. 7, the optical measurement apparatus 1 inserts the distal end of the measurement probe 3 into the container 111, in which the second medium W2 is accommodated, to execute the third measurement process of measuring the second medium W2, before measuring the specimen S1. In this case, the detection unit 23 outputs a signal intensity "c" (third detection intensity "c") detected, to the background information recording unit 261 of the recording unit 26. When the third measurement process is performed, quantity of light of the light source unit 21 may change in comparison with the third measurement process.

Next, the measurement process of the specimen S1 is described. As illustrated in FIG. 8, the optical measurement apparatus 1 contacts the distal end of the measurement probe 3 with the specimen S1 to perform the measurement. In this case, the detection unit 23 detects an intensity "I" of the returned light of the illumination light that has been irradiated by the illumination fiber 31 and reflected and/or scattered from the specimen S1. The intensity "I" of the returned light includes a background intensity "B" generated inside the measurement probe 3. Therefore, the calculation unit 271 calculates returned light "S" of the illumination light from only the specimen S1, by using Equation (1) below.

$$S = I - B \qquad\qquad (1)$$

The background intensity "B" is assumed to not include electric noise or the like, of which the detection unit 23 is the source.

The background intensity "B" is expressed by Equation (2) below using the signal strength "a" of the first measurement process, the signal intensity "b" of the second measurement process, and the signal strength "c" of the third measurement process, which have been recorded in the background information recording unit 261.

$$B = (a/b) \times c \qquad (2)$$

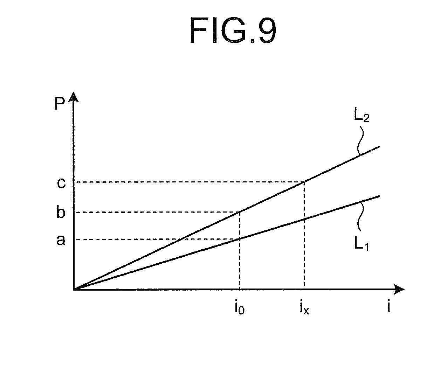

The relational equation of Equation (2) is described in detail using FIG. 9.

FIG. 9 is a diagram that illustrates a relation between incident light intensity of light incident on the measurement probe 3 and signal intensity. Further, in FIG. 9, the horizontal axis "i" represents the incident light intensity of light incident on the measurement probe 3, and the vertical axis "P" represents the signal intensity. Further, a straight line $L_1$ represents the signal intensity measured when the measurement probe 3 is contacted with the first medium W1, and a straight line $L_2$ represents the signal intensity measured when the measurement probe 3 is contacted with the second medium W2. The straight line $L_1$ is assumed to be represented as $P = C_1(i)$, and the straight line $L_2$ is assumed to be represented as $P = C_2(i)$.

As illustrated in FIG. 9, if an incident light intensity of light incident on the measurement probe 3 in each of the first measurement process and the second measurement process is "$i_0$", from a straight line $L_w$ and a straight line $L_a$, the following Equation (3) and Equation (4) hold true.

$$P = C_1(i_0) = a \qquad (3)$$

$$P = C_2(i_0) = b \qquad (4)$$

Further, if an incident light intensity of light incident on the measurement probe 3 in the third measurement process is "$i_x$", the following Equation (5) holds true.

$$P = C_2(i_x) = c \qquad (5)$$

Further, since the specimen S1 and the first medium W1 have the same refractive indexes, the background intensity "B" in the measurement process of the specimen S1 is represented as follows.

$$B = C_1(i_x) \qquad (6)$$

Since the straight line $L_2$ and the straight line $L_1$ satisfy $C_2(0) = 0$ and $C_1(0) = 0$, the following holds true.

$$B = (C_1(i_0)/C_2(i_0)) \times C_2(i_x)$$
$$= (a/b) \times c \qquad (7)$$

According to Equation (7), the calculation unit 271 calculates the background intensity "B" using the first detection intensity "a" obtained in the first measurement process, the second detection intensity "b", and the third detection intensity "c" obtained in the third measurement process.

According to the above described first embodiment of the present invention, from the product of the third detection intensity and the value of the ratio of the first detection intensity to the second detection intensity, the value being recorded in the background information recording unit 261, the background intensity used in correcting the returned light of the illumination light from the specimen S1 is calculated. Accordingly, when measuring body tissue, by omitting measurement of background light using water or a liquid, operations of a calibration process involved in measurement are able to be facilitated. That is, in measuring reflection and scattering of the body tissue, the background intensity of the background light is readily obtainable in a state in which the measurement probe is in contact with the body tissue.

Further, according to the first embodiment of the present invention, when the measurement probe 3 irradiates the illumination light to the specimen S1, the calculation unit 271 subtracts the background intensity from the detection intensity of the returned light of the illumination light from the specimen S1 detected by the detection unit 23, and thus accurate measurement values of the specimen S1 are obtainable.

In the first embodiment of the present invention, a plurality of detection fibers may be provided in the measurement probe. In that case, a plurality of detection units corresponding respectively to the plurality of detection fibers are provided in the main body unit 2.

Further, in the first embodiment of the present invention, although the measurement probe 3 is detachable with respect to the main body unit 2, the measurement probe and the main body unit may be integrally formed with each other.

(Second Embodiment)

Next, a second embodiment of the present invention will be described. In the second embodiment, a calibration process is executed for each measurement probe attached to a main body unit. Therefore, hereinafter, after a configuration of an optical measurement apparatus according to the second embodiment is described, a calculation process including a calibration process executed by the optical measurement apparatus is described. The same components as those of the optical measurement apparatus according to the above described first embodiment will be described being appended with the same reference signs.

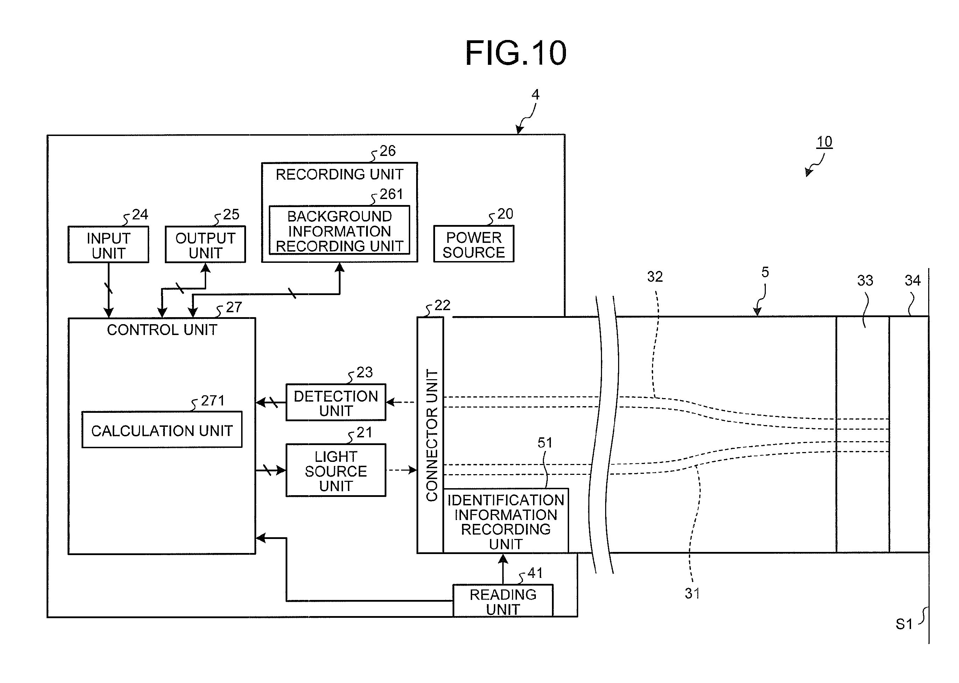

FIG. 10 is a block diagram that illustrates a configuration of an optical measurement apparatus according to a second embodiment of the present invention. An optical measurement apparatus 10 illustrated in FIG. 10 includes a main body unit 4 and a measurement probe 5.

First, the main body unit 4 is described. The main body unit 4 includes the power source 20, the light source unit 21, the connector unit 22, the detection unit 23, the input unit 24, the output unit 25, the recording unit 26, the control unit 27, and a reading unit 41.

The reading unit 41 reads, from an identification information recording unit 51 provided on a proximal end side of a later described measurement probe 5, identification information including specific information (ID information)that identifies the measurement probe 5, background information, and observation information indicating a measureable specimen S1 and an applicable site, and outputs the read identification information to the recording unit 26. The reading unit 41 is realized by using an IC reader-writer, a bar code reader, or the like.

Next, the measurement probe 5 is described. The measurement probe 5 includes the illumination fiber 31, the detection fiber 32, the fiber holding portion 33, the rod lens 34, and the identification information recording unit 51.

The identification information recording unit 51 records therein the identification information including the specific information (ID information) identifying the measurement probe 5, the background information, and the observation information indicating the measurable specimen S and the applicable site. The identification information recording unit 51 is realized by using an IC chip, a bar code, or the like. The background information may be respective values of the first detection intensity detected by the first measurement process executed beforehand, and the second detection intensity detected by the second measurement process. Instead, it may be a value of a ratio therebetween.

In the optical measurement apparatus 10 configured as above, the calculation unit 271 calculates a measurement value of the specimen S1 for which background light has been corrected, by performing the above described calibration process based on the identification information of the measurement probe 5 read by the reading unit 41 every time a different measurement probe 5 is connected to the main body unit 4.

According to the above described second embodiment of the present invention, because measurement of background light is omittable when measuring the specimen S1, a calibration process involved in the measurement of the specimen S1 is able to be readily performed.

In the second embodiment of the present invention, a seal member, on which background information related to background light is written, may be stuck onto the measurement probe 5 and a user may input the background information on the stuck seal member by using the input unit 24.

Further, in the second embodiment of the present invention, the first measurement process and second measurement process may be performed for every measurement probe 5, and the first detection intensity "a" and second detection intensity "b" may be recorded in the background information recording unit 261, and when the specimen S1 is measured, the background information of the measurement probe 5 to be used may be used via the input unit 24.

(Third Embodiment)

Next, a third embodiment of the present invention will be described. In the third embodiment, characteristics of the object to be measured are estimated by measuring a spectrum of the object to be measured. Therefore, hereinafter, after a configuration of an optical measurement apparatus according to the third embodiment is described, a calculation process including a calibration process executed by the optical measurement apparatus is described. The same components as those of the optical measurement apparatus according to the above described first embodiment will be described being appended with the same reference signs.

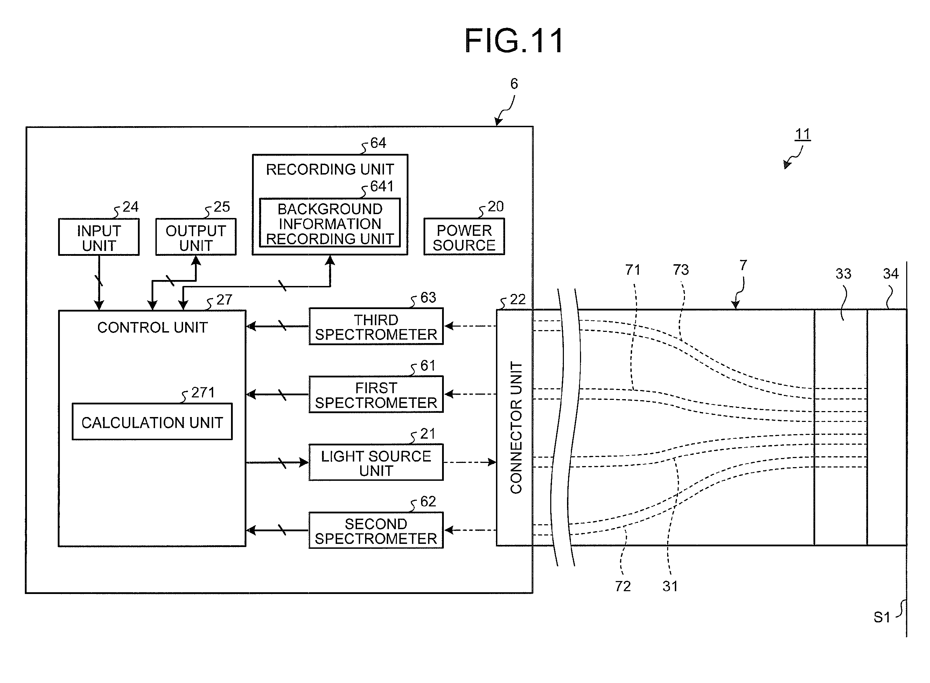

FIG. 11 is a block diagram illustrating the configuration of the optical measurement apparatus according to the third embodiment of the present invention. An optical measurement apparatus 11 illustrated in FIG. 11 includes: a main body unit 6 that performs optical measurement with respect to a specimen S1 such as body tissue, which is a scatterer, and detects characteristics of the specimen S1; and a measurement probe 7 that is detachable with respect to the main body unit 6, inserted into a subject, and of a disposable type.

First, the main body unit 6 is described. The main body unit 6 includes the power source 20, the light source unit 21, the connector unit 22, the input unit 24, the output unit 25, the control unit 27, a first spectrometer 61, a second spectrometer 62, a third spectrometer 63, and a recording unit 64.

The first spectrometer 61 detects a spectrum of returned light of illumination light irradiated from the measurement probe 7 and reflected and/or scattered from the specimen S1, and outputs a result of this detection to the control unit 27.

The second spectrometer 62 is realized by the same configuration as the first spectrometer 61, detects a spectrum of returned light of the illumination light irradiated from the measurement probe 7 and reflected and/or scattered from the specimen S1, and outputs a result of this detection to the control unit 27.

The third spectrometer 63 is realized by the same configuration as the first spectrometer 61, detects a spectrum of returned light of the illumination light irradiated from the measurement probe 7 and reflected and/or scattered from the specimen S1, and outputs a result of this detection to the control unit 27.

The recording unit 64 is realized by using a volatile memory, a non-volatile memory, and the like, and records therein various programs for operating the main body unit 6, and various data, various parameters, and the like used in an optical measurement process. Further, the recording unit 64 records therein measurement results of the specimen S1 by the main body unit 6. Further, the recording unit 64 has a background information recording unit 641.

The background information recording unit 641 records therein respective values or a value of a ratio therebetween, of a first spectrum $a(\lambda)$ detected for each wavelength not including returned light of the illumination light from the first medium W1 having the same refractive index as the body tissue when the illumination light is irradiated by the measurement probe 7 to the first medium W1, and a second spectrum $b(\lambda)$ detected for each wavelength not including returned light of the illumination light from the second medium W2 when the illumination light is irradiated by the measurement probe 7 to the second medium W2.

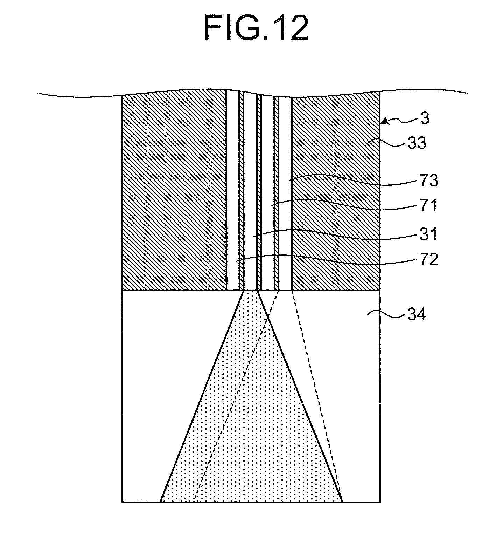

Next, the measurement probe 7 is described. FIG. 12 is a diagram that schematically illustrates a cross section of a distal end of the measurement probe 7, the cross section being cut along a longitudinal direction thereof.

The measurement probe 7 illustrated in FIGS. 11 and 12 includes the illumination fiber 31, the fiber holding portion 33, the rod lens 34, a detection fiber 71 (first light receiving channel C1), a second detection fiber 72 (second light receiving channel C2), and a third detection fiber 73 (third light receiving channel C3).

The first detection fiber 71 is realized by using an optical fiber, and detects (optically receives) via the rod lens 34 and propagates to the first spectrometer 61, the returned light of the illumination light reflected and/or scattered from the specimen S1.

The second detection fiber 72 is realized by using an optical fiber, and detects via the rod lens 34 and propagates to the second spectrometer 62, the returned light of the illumination light reflected and/or scattered from the specimen S1.

The third detection fiber 73 is realized by using an optical fiber, and detects via the rod lens 34 and propagates to the third spectrometer 63, the returned light of the illumination light reflected and/or scattered from the specimen S1.

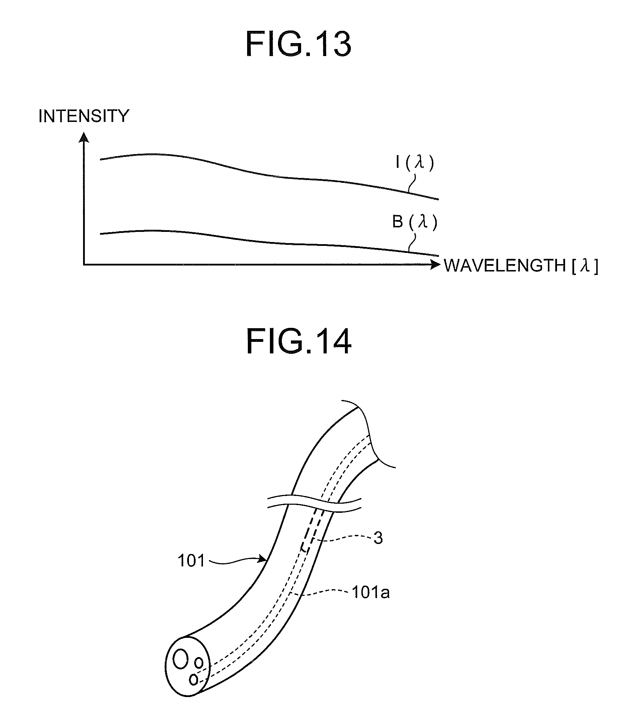

A calculation process on the specimen S1 executed by the calculation unit 271 of the optical measurement apparatus 11 configured as above is described. FIG. 13 is a diagram that illustrates a relation between a measurement spectrum when the optical measurement apparatus 11 measures the specimen S1 and a measurement spectrum of background light.

First, the optical measurement apparatus 11 measures a third spectrum $c(\lambda)$ by performing the above described third measurement process, before measuring the specimen S1. In this case, the calculation unit 271 calculates a spectrum $B(\lambda)$ of background light for each of the first detection fiber 71, the second detection fiber 72, and the third detection fiber 73 using Equation (8) below.

$$B(\lambda) = ((a(\lambda)/b(\lambda)) \times c(\lambda) \qquad (8)$$

Subsequently, the optical measurement apparatus 11 measures a spectrum $I(\lambda)$ of the specimen S1. In this case, the calculation unit 271 calculates a spectrum $S(\lambda)$ of the specimen S1 by subtracting the spectrum $B(\lambda)$ of the background light from the spectrum $I(\lambda)$ of the specimen S1, using Equation (9) below (see FIG. 13).

$$S(\lambda) = I(\lambda) - B(\lambda)$$
$$= I(\lambda) - (a(\lambda)/b(\lambda)) \times c(\lambda) \qquad (9)$$

Equation (8) is substituted into a second term.

According to the above described third embodiment of the present invention, when measuring the specimen S1, measurement of a spectrum of background light using water or a liquid is omittable, and thus a calibration process involved in the measurement is able to be facilitated.

(Other Embodiment)

In another embodiment of the present invention, the third measurement process may be performed in a treatment tool channel (inside an endoscope forceps opening). Specifically, as illustrated in FIG. 14, by using the treatment tool channel 101a of the endoscope 101 as a space for absorbing light, without providing the container 111 that accommodates the second medium W2, the third measurement process is executable.

Reference Signs List

| | |
|---|---|
| 1, 10, 11 | Optical measurement apparatus |
| 2, 4, 6 | Main body unit |
| 3, 5, 7 | Measurement probe |
| 20 | Power source |
| 21 | Light source unit |
| 22 | Connector unit |
| 23 | Detection unit |
| 24 | Input unit |
| 25 | Output unit |
| 26, 64 | Recording unit |
| 27 | Control unit |
| 31 | Illumination fiber |
| 32 | Detection fiber |
| 33 | Fiber holding portion |
| 34 | Rod lens |
| 41 | Reading unit |
| 51 | Identification information recording unit |
| 61 | First spectrometer |
| 62 | Second spectrometer |
| 63 | Third spectrometer |
| 71 | First detection fiber |
| 72 | Second detection fiber |
| 73 | Third detection fiber |
| 261, 641 | Background information recording unit |
| 271 | Calculation unit |

**Claims**

1. An optical measurement apparatus, comprising:

   a measurement probe that irradiates a specimen with illumination light and optically receives returned light of the illumination light reflected and/or scattered from the specimen;
   a detection unit that detects the returned light optically received by the measurement probe;
   a recording unit that records therein a value of a ratio of a first detection intensity to a second detection intensity, the first detection intensity being an intensity of the returned light detected when a first medium having a refractive index that is the same as that of the specimen is irradiated with the illumination light by the measurement probe and detected in a state of not including the returned light from the first medium, and the second detection intensity being an intensity of the returned light detected when a second medium is irradiated with the illumination light by the measurement probe and detected in a state of not including the returned light from the second medium; and

a calculation unit that calculates, from a product of a third detection intensity and the value of the ratio, a background intensity of background light to be used in correcting the returned light from the specimen, the third detection intensity being an intensity of the returned light detected when the second medium is irradiated with the illumination light by the measurement probe and detected in a state of not including the returned light from the second medium.

2. The optical measurement apparatus according to claim 1, wherein the calculation unit subtracts the background intensity from a detection intensity of the returned light from the specimen detected by the detection unit when the measurement probe irradiates the specimen with the illumination light.

3. The optical measurement apparatus according to claim 2, wherein the detection unit has a spectrometer, and the first detection intensity, the second detection intensity, and the third detection intensity are values detected for each wavelength spectrally dispersed by the spectrometer.

4. The optical measurement apparatus according to any one of claims 1 to 3, wherein the first medium is water.

5. The optical measurement apparatus according to any one of claims 1 to 4, wherein the second medium is air.

6. The optical measurement apparatus according to any one of claims 1 to 5, wherein the measurement probe is detachable to a main body unit of the optical measurement apparatus.

7. The optical measurement apparatus according to any one of claims 1 to 6, wherein the specimen is body tissue.

8. The optical measurement apparatus according to any one of claims 1 to 7, wherein an absolute value of a difference between the refractive index of the specimen and the refractive index of the first medium is equal to or less than 0.1.

9. The optical measurement apparatus according to any one of claims 1 to 8, wherein
the first detection intensity is measured with respect to the first medium accommodated in a container provided with, on an inner surface thereof, a light absorption member that absorbs light, and
the second detection intensity is measured with respect to the second medium accommodated in the container.

10. A calibration method executed by an optical measurement apparatus that includes a measurement probe, which irradiates a specimen with illumination light and optically receives returned light of the illumination light reflected and/or scattered from the specimen, and that performs optical measurement of the specimen, the calibration method comprising the steps of:

calculating a background intensity of background light to be used in correcting the returned light from the specimen, from a product of a value and a third detection intensity, the value being of a ratio of a first detection intensity to a second detection intensity, the first detection intensity being an intensity of the returned light detected when a first medium having a refractive index that is the same as that of the specimen is irradiated with the illumination light by the measurement probe and detected in a state of not including the returned light from the first medium, the second detection intensity being an intensity of the returned light detected when a second medium is irradiated with the illumination light by the measurement probe and detected in a state of not including the returned light from the second medium, and the third detection intensity being an intensity of the returned light detected when the second medium is irradiated with the illumination light by the measurement probe and detected in a state of not including the returned light from the second medium; and
subtracting the background intensity from a detection intensity of the returned light from the specimen.

**FIG.1**

# FIG.2

# FIG.3

# FIG.4

## FIG.5

## FIG.6

# FIG.7

# FIG.8

# FIG.9

# FIG.10

# FIG.11

EP 2 759 247 A1

# FIG.12

# FIG.13

INTENSITY

I($\lambda$)

B($\lambda$)

WAVELENGTH [$\lambda$]

# FIG.14

101

3

101a

<div align="center">

**INTERNATIONAL SEARCH REPORT**

</div>

| International application No. |
|---|
| PCT/JP2012/073948 |

**A. CLASSIFICATION OF SUBJECT MATTER**
*A61B1/00*(2006.01)i, *G01N21/27*(2006.01)i

According to International Patent Classification (IPC) or to both national classification and IPC

**B. FIELDS SEARCHED**

Minimum documentation searched (classification system followed by classification symbols)
A61B1/00, G01N21/27

Documentation searched other than minimum documentation to the extent that such documents are included in the fields searched
Jitsuyo Shinan Koho        1922-1996   Jitsuyo Shinan Toroku Koho   1996-2012
Kokai Jitsuyo Shinan Koho   1971-2012   Toroku Jitsuyo Shinan Koho   1994-2012

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 07-167773 A (Ohmeda Inc.),<br>04 July 1995 (04.07.1995),<br>paragraphs [0004], [0006] to [0013]; fig. 1 to 4<br>& US 5365925 A        & EP 638280 A3 | 1-10 |
| A | JP 2008-089565 A (National University Corporation Toyohashi University of Technology),<br>17 April 2008 (17.04.2008),<br>paragraphs [0012] to [0014]<br>(Family: none) | 1-10 |
| A | JP 2010-227256 A (Olympus Corp.),<br>14 October 2010 (14.10.2010),<br>paragraphs [0014] to [0121]; fig. 1<br>& US 2010/0245551 A1 | 1-10 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 October, 2012 (09.10.12) | 16 October, 2012 (16.10.12) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
|---|
| PCT/JP2012/073948 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2009-537014 A  (Northwestern University),<br>22 October 2009 (22.10.2009),<br>entire text; all drawings<br>& US 2008/0037024 A1     & US 2009/0009759 A1<br>& EP 2029961 A            & WO 2007/133684 A2<br>& CA 2651799 A            & CN 101548153 A<br>& AU 2007249858 A | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090009759 A

**Non-patent literature cited in the description**

- 41. *Phys. Med. Biol.,* 1996, 369
- *Opt. Lett.,* 2006, vol. 31, 2759